# EUROPEAN PATENT SPECIFICATION

(11) **EP 2 695 350 B1**
(45) Date of publication and mention of the grant of the patent: **04.12.2019**
(21) Application number: 12768542.8
(22) Date of filing: 05.04.2012
(51) Int. Cl.: H04L 29/04

(54) **MEDICAL SENSING COMMUNICATION METHOD**
MEDIZINISCHES ABTAST- UND KOMMUNIKATIONSVERFAHREN
PROCÉDÉ DE COMMUNICATION DE DÉTECTION MÉDICALE

(30) Priority: 08.04.2011 US 201161473625 P
(43) Date of publication of application: 12.02.2014
(73) Proprietor: Volcano Corporation, San Diego, CA 92130 (US)
(72) Inventor: SPENCER, Jason, Rocklin, California 95765 (US); LITZZA, Gerald L., Sacramento, California 95864 (US); LINDSAY, Craig A., Sacramento, California 95831 (US); GLOVER, Richard, London W5 5NW (GB)
(74) Representative: de Haan, Poul Erik
(86) International application number: PCT/US2012/032345
(87) International publication number: WO 2012/138874

(56) References cited:
- KR-A- 20030 025 748
- KR-A- 20030 074 520
- US-A1- 2002 044 059
- US-A1- 2009 030 405
- US-A1- 2009 312 638
- US-A1- 2010 169 123
- US-A1- 2010 249 540
- US-A1- 2011 015 504

## Description

### TECHNICAL FIELD

Embodiments of the present disclosure relate generally to the field of medical devices and, more particularly, to a medical communication system and associated methods of use.

### BACKGROUND

This application claims the benefit of U.S. provisional patent application 61/473,625, filed April 8, 2011, entitled "MEDICAL SENSING COMMUNICATION SYSTEM AND METHOD". Innovations in diagnosing and verifying the level of success of treatment of disease have migrated from external imaging processes to internal diagnostic processes. In particular, diagnostic equipment and processes have been developed for diagnosing vasculature blockages and other vasculature disease by means of ultra-miniature sensors placed upon the distal end of a flexible elongate member such as a catheter, or a guide wire used for catheterization procedures. For example, known medical sensing techniques include angiography, intravascular ultrasound (IVUS), forward looking IVUS (FL-IVUS), fractional flow reserve (FFR) determination, a coronary flow reserve (CFR) determination, optical coherence tomography (OCT), trans-esophageal echocardiography, and image-guided therapy. Each of these techniques may be better suited for different diagnostic situations. To increase the chance of successful treatment, health care facilities may have a multitude of imaging and sensing modalities on hand in a catheter lab during a procedure. However, each imaging modality in a catheter lab traditionally requires its own special-purpose diagnostic equipment. For instance, an imaging modality may require a catheter, a patient isolation module (PIM), a user control interface, a display, a specialized power unit, and a processing unit such as a customized personal computer. Traditionally, all of this equipment is located in the catheter room itself during a procedure and depends on a substantial wiring infrastructure for network connectivity and dependable power. Physical space is typically at a premium in catheter labs and each additional imaging modality employed in a catheter lab complicates the pre-procedure setup and limits the movement of health care professionals during procedures. Additionally, known problems arise when attempting to locate portions of a diagnostic system outside of a catheter lab. For instance, data transmitted between a PIM and a processing unit may degrade as the distance between the two increases. Similarly, traditional long-distance communication links do not support the bandwidth required for modern cardiovascular imaging techniques.

While the existing devices and methods have been generally adequate for their intended purposes, they have not been entirely satisfactory in all respects. The medical sensing systems and associated methods of the present disclosure overcome one or more of the shortcomings of the prior art.

In US 2002/0044059, a patient monitoring system comprises a computer, an input device coupled to the computer and configured to input patient information, and first and second display screens coupled to the computer. The computer displays a first portion of the patient information on the first display screen and a second portion of the patient information on the second display screen.

According to US 2010/0249540, systems and methods for monitoring multiple physiological parameters made available by multiple sensors positioned on a patient are provided. The system may include a plurality of sensors, a multi-parameter monitor and a plurality of displays. The monitor may include a plurality of sensor interfaces, a multi-parameter processor and an output interface. The sensors connect to the sensor interfaces and generate physiological signals that are transmitted to the processor for processing. The processed signals are transmitted to at least one display for viewing by medical personnel. The sensor interfaces are of like kind and provide easy connection and disconnection for exchange of sensor types, such as ECG, oximetry, body temperature and NIBP. The monitor may communicate with the plurality of displays. When a patient is transported from one location to another, the monitor can be transported with the patient and operatively link to displays in the new location thus eliminating the need to transport multiple devices or machines from one location to another.

### SUMMARY

The present invention is defined in the claims. In one exemplary aspect, the present disclosure is directed to a patient communication system including a housing, a first socket disposed on the housing and configured to receive first medical data associated with a first modality from a first medical sensing device communicatively coupled thereto, and a controller disposed within the housing and configured to digitize the first medical data if it is received by the first socket in analog form. The system also includes a network communication module disposed within the housing and configured to transmit the digitized first medical data onto a data network and a first power module disposed within the housing and configured to provide a first dynamic amount of power to the first medical sensing device through the first socket based on the power requirements of the first medical sensing device.

In another exemplary aspect, the present disclosure is directed to a method of collecting medical sensing data with a patient communication system. The method includes receiving, through a first socket disposed on a housing of the patient communication system, first medical data associated with a first modality from a first medical sensing device communicatively coupled to the first socket and digitizing, with a controller disposed in the housing, the first medical data if it is received in analog form. The method also includes transmitting, with a network communication module disposed within the housing, the digitized first medical data onto a data network and providing a first dynamic amount of power, with a first power module disposed within the housing, to the first medical sensing device through the first socket.

In yet another exemplary aspect, the present disclosure is directed to a patient communication system. The system includes a control module configured to receive first medical data associated with a first modality from a first body sensing device and second medical data associated with a second modality from a second body sensing device. The system also includes a first power module configured to dynamically supply a first amount of power to the first body sensing device based on a power requirement of the first body sensing device and a second power module configured to dynamically supply a second amount of power to the second body sensing device based on a power requirement of the second body sensing device. Further, the system includes a communication module operable to transmit the first and second medical data to a data network.

In another exemplary aspect, the present disclosure is directed to a method of using a patient communication system. The method of using a patient communication system includes coupling a first body sensing device to a first patient isolation module, the first body sensing device including a first sensor disposed thereon and coupling the first patient isolation module to a hub, the hub communicatively coupled to a data network. The method further includes coupling a second body sensing device to a second patient isolation module, the second body sensing device including a second sensor disposed thereon and coupling the second patient isolation module to the hub. Further, the method includes utilizing the first sensor to collect first medical characteristic data associated with a patient, the collecting including transmitting the first medical characteristic data to the hub and also utilizing the second sensor to collect second medical characteristic data associated with the patient, the collecting including transmitting the second medical characteristic data to the hub. The method also includes transmitting, with the hub, the first and second medical characteristic data to the data network and receiving, at a user interface, processed first and second medical characteristic data from the data network, the user interface being communicatively coupled to the hub.

### BRIEF DESCRIPTION OF THE DRAWINGS

Figure 1 is a schematic drawing depicting a medical sensing communication system including a bedside utility box according to one embodiment of the present disclosure.
Figure 2 is a diagrammatic perspective view of the bedside utility box of Figure 1.
Figure 3 is a functional block diagram of an embodiment of a bedside utility box.
Figure 4 is a functional block diagram of an aspect of the medical sensing communication system that includes a software framework executing on a bedside utility box.
Figure 5 is a schematic drawing depicting a medical sensing communication system according to another embodiment of the present disclosure.
Figure 6 is a functional block diagram of an exemplary embodiment of an aspect of the medical sensing communication system of Figure 5, specifically, a patient isolation module.

### DETAILED DESCRIPTION

For the purposes of promoting an understanding of the principles of the present disclosure, reference will now be made to the embodiments illustrated in the drawings, and specific language will be used to describe the same. It will nevertheless be understood that no limitation of the scope of the disclosure is intended. Any alterations and further modifications in the described devices, instruments, methods, and any further application of the principles of the disclosure as described herein are contemplated as would normally occur to one skilled in the art to which the disclosure relates. In particular, it is fully contemplated that the features, components, and/or steps described with respect to one embodiment may be combined with the features, components, and/or steps described with respect to other embodiments of the present disclosure.

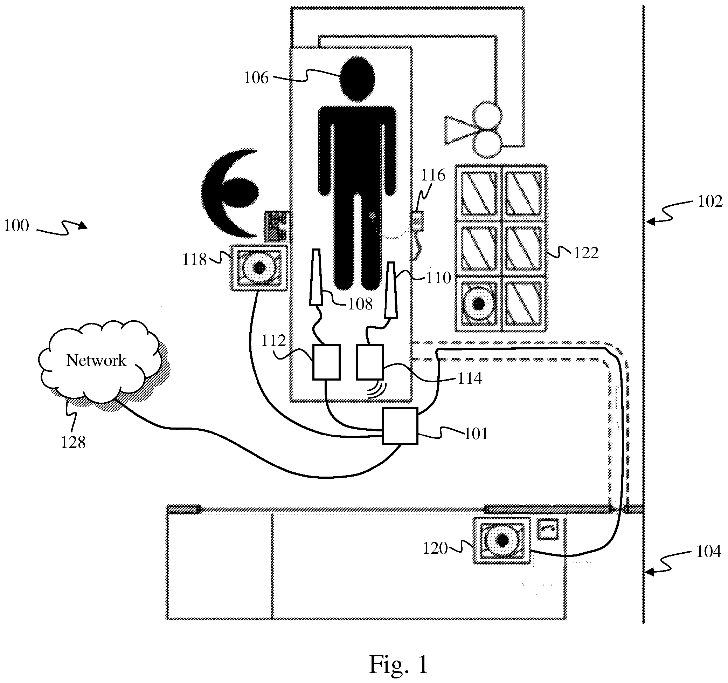

Figure 1 is a schematic drawing depicting a medical sensing communication system 100 including a bedside utility box (BUB) 101. The medical sensing communication system 100 is a network-connected, data collection solution for multiple modality medical sensing. Generally, in the system 100, the BUB 101 is a central hub that interconnects a plurality of medical sensing-related tools and facilitates communication between the tools and a data network. In one embodiment, the communication system 100 may be utilized to collect data from medical sensing devices and transmit it to remote computing resources, where it is processed and returned. WO2012154335, entitled "MULTI-MODALITY MEDICAL SENSING SYSTEM AND METHOD" and filed on April 8, 2011, discloses a computing resource capable of processing multi-modality medical sensing data and is hereby incorporated by reference in its entirety.

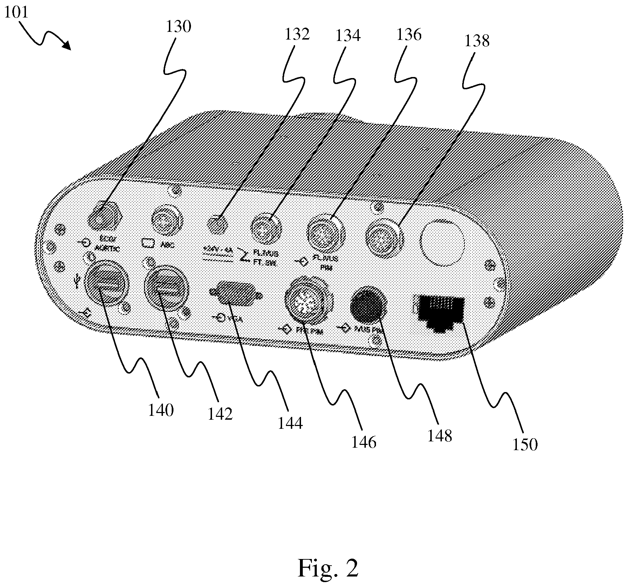

In the illustrated embodiment, the medical sensing communication system 100 is deployed in a catheter lab 102 having a control room 104. The catheter lab 102 includes a sterile field but its associated control room 104 may or may not be sterile depending on the requirements of a procedure and/or health care facility. The catheter lab and control room may be used to perform on a patient any number of medical sensing procedures such as angiography, intravascular ultrasound (IVUS), virtual histology (VH), forward looking IVUS (FL-IVUS), intravascular photoacoustic (IVPA) imaging, a fractional flow reserve (FFR) determination, a coronary flow reserve (CFR) determination, optical coherence tomography (OCT), computed tomography, intracardiac echocardiography (ICE), forward-looking ICE (FLICE), intravascular palpography, transesophageal ultrasound, or any other medical sensing modalities known in the art. For example, in catheter lab 102 a patient 106 may be undergoing a multi-modality procedure, in which IVUS data will be collected with an IVUS catheter 108 and OCT data will be collected with an OCT catheter 110. The IVUS catheter 108 may include one or more sensors such as a phased-array transducer. In some embodiments, the IVUS catheter 108 may be capable of multi-modality sensing such as IVUS and IVPA sensing. The OCT catheter 110 may include one or more optical sensors. The communication system 100 includes a number of interconnected medical sensing-related tools in the catheter lab 102 and control room 104 to facilitate this multi-modality workflow procedure, including an IVUS patient isolation module (PIM) 112, an OCT PIM 114, an electrocardiogram (ECG) device 116, a bedside control surface 118, a control room control surface 120, and a boom display 122. The BUB 101 in the catheter lab 102 interconnects these medical sensing-related tools and communicatively couples them to a data network 128. That is, the BUB 101 is a central hub through which the tools in the catheter lab 102 and control room 104 connect to the data network 128. In the illustrated embodiment, the data network 128 is TCP/IP-based local area network (LAN), however in other embodiments, it may utilize a different protocol such as Synchronous Optical Networking (SONET), or may be a wide area network (WAN). Further, in another embodiment, the data network 128 may be a data bus network, such as a Universal Serial Bus (USB) network, for connecting BUB 101 to a host controller with computing resources. The BUB 101 will be described in greater detail in association with Figures 2-4.

In the illustrated embodiment of Figure 1, the IVUS catheter 108, PIM 112, and the BUB 101 together may be considered a patient communication system that is operable to receive medical sensing data collected from the patient 106 by the IVUS catheter 108 and to transmit the received data onto the data network 128. As shown, the BUB 101 is communicatively coupled to the IVUS patient isolation module (PIM) 112 via a wired connection such as a standard copper link or a fiber optic link, and the IVUS PIM is, in turn, coupled to the IVUS catheter 108 via a similar wired connection. In alternative embodiments, however, the BUB-PIM connection and/or the PIM-catheter connection may be wired or wireless. In one embodiment, the PIM 112 includes an analog to digital (A/D) converter and transmits digital data to the BUB 101, however, in other embodiments the PIM transmits analog data to the BUB. Further, in some embodiments, the PIM 112 and BUB 101 communicate with a standardized data transmission protocol, such as Synchronous Optical Networking (SONET). Further, the PIM 112 provides power to data collection sensors disposed on the catheter 108 via its connection to BUB 101. Typically, different sensory instruments require different amounts of power, and thus their associated PIMs may draw different amounts of power from the BUB 101. As discussed later, the BUB 101 is operable to dynamically provide the appropriate amount of power to IVUS PIM 112 via the wired connection.

Further, the OCT catheter 110 and PIM 114 may also be considered a part of the patient communication system that includes PIM 112 and BUB 101. With the OCT catheter 110 and PIM 114 the patient communication system is further operable to receive medical sensing data collected by the OCT catheter 110 and transmit the received data onto the data network 128. In the illustrated embodiment, the OCT PIM 114 is communicatively coupled to BUB 101 via a wireless connection, but in alternative embodiments, may be communicatively coupled via a wired connection. In one embodiment, the PIM 114 includes an A/D converter and transmits digital data to the BUB 101, however, in other embodiments the PIM transmits analog data to the BUB. Further, in some embodiments, the PIM 114 and BUB 101 communicate with a standardized data transmission protocol, such as SONET. In some embodiments, the PIM 114 may include a battery to power catheter 110, may draw power from a wired power connection, or may be wirelessly powered.

For convenience purposes, the PIMs 112 and 114 may hang from the patient table or may be placed in another location near the patient. Although two PIMs are depicted as communicatively coupled to the BUB 101, additional PIMs associated with different medical sensing modalities may be connected to BUB 101. Any such additional PIMs may communicate with the BUB 101 concurrently with the PIMs 112 and 114. Still further, a single PIM may support multiple modalities, such as IVUS and IVPA. Additionally, in some embodiments, such as those in which patient data is collected using angiography, one of the illustrated PIMs may be replaced with a C-arm. In such embodiments, the C-arm may act as the power and data intermediary between the angiography sensors and the network 128. Still further, in another embodiment, the medical sensing communication system 100 may include an adapter device to serve as an intermediary between a third-party system such as an angiography system and the BUB 101. Such an adaptor device may transform data in a proprietary third-party format into a format usable by the system 100. U.S. Patent Application Publication No. US 2007/0232933, entitled "Component-Based Catheter Lab Intravascular Ultrasound System," discloses a component-based IVUS system that includes a PIM.

As mentioned above, the ECG device 116 is also communicatively coupled to BUB 101 via a wired or wireless connection. The ECG device 116 is operable to transmit electrocardiogram signals from patient 106 to the BUB 101. In some embodiments, the BUB 101 may be operable to synchronize data collection with the catheters 108 and 110 using the ECG signals from the ECG 116.

The bedside control surface 118 is also communicatively coupled to the BUB 101 and provides user control of the particular medical sensing modality (or modalities) being used to diagnose the patient 106. In the current embodiment, the bedside control surface 118 is a touch screen that provides user controls and diagnostic images on a single surface. In alternative embodiments, however, the bedside control surface 118 may include both a non-interactive display and separate controls such as physical buttons and/or a joystick. In the illustrated embodiment, the bedside control surface 118 and BUB 101 communicate over a wired connection such as a standard copper link or a fiber optic link but, alternatively, the control surface 118 and BUB 101 may communicate wirelessly. Further, in some embodiments, the bedside control surface 118 may also be communicatively coupled directly to one or both of PIMs 112 and 114. The bedside control surface 118 includes an integrated processing unit to drive a graphical user interface (GUI)-based workflow presented on the touch screen. In an exemplary embodiment, the particular GUI-based workflow presented by the bedside control surface 118 depends on the medical sensing modality being used to diagnose the patient 106. To this end, the bedside control surface 118 is capable of displaying multiple GUI-based workflows, each corresponding to a particular sensor or imaging modality or simultaneous combination thereof. The bedside control surface 118 is further operable to display co-registration GUI-based workflows, for example, to integrate sensing data collected by catheters 108 and 110. An API-based software framework executing on the bedside control surface 118 manages the multiple workflows. WO2012138872, entitled "Distributed Medical Sensing System and Method" and filed on April 8, 2011, discloses a bedside control surface that executes GUI-based workflows using a software framework and is hereby incorporated by reference in its entirety.

The control room control surface 120 in the control room 104 is also communicatively coupled to the BUB 101 and, as shown in Figure 1, is adjacent to catheter lab 102. In the illustrated embodiment, the control room control surface 120 and BUB 101 communicate over a wired connection such as a standard copper link or a fiber optic link but, alternatively, the control surface 120 and BUB 101 may wirelessly communicate. In the current embodiment, the control room control surface 120 is similar to the bedside control surface 118 in that it includes a touch screen, integrated processing unit, and multitude of GUI-based workflows corresponding to different medical sensing modalities. During a procedure, however, the control room control surface 120 may be used to carry out a different aspect of the procedure's workflow than the bedside control surface 118. In alternative embodiments, the control room control surface 120 may include a non-interactive display and standalone controls such as a mouse and keyboard. Further, the processing unit of the control room control surface 120 may be more powerful than the processing unit of the bedside control surface 118.

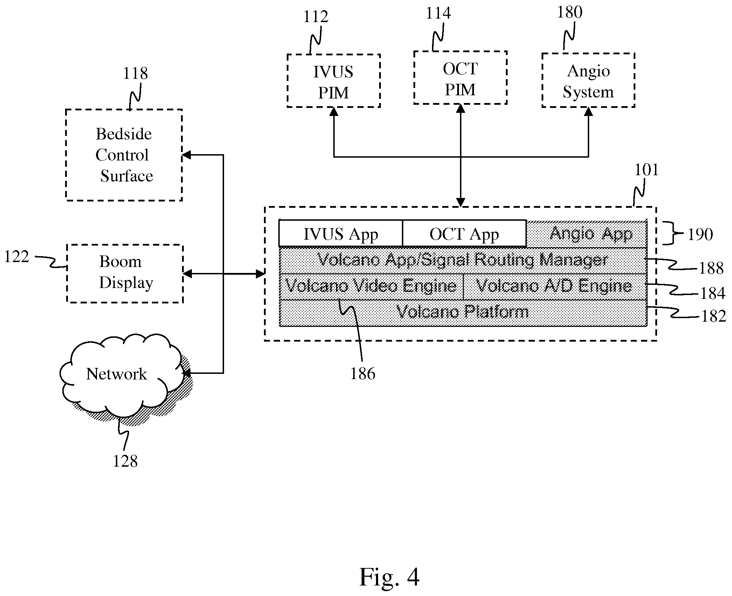

The system 100 further includes the boom display 122. The boom display 122 may include an array of monitors, each capable of displaying different information associated with a medical sensing procedure. For example, during an IVUS procedure, one monitor in the boom display 122 may display a tomographic view and one monitor may display a sagittal view. In an embodiment as described in Figure 4, the boom display 122 may be coupled directly to and driven by the BUB 101. In other embodiments, the boom display may also be operable to receive image data from the bedside control surface 118 or the control room control surface 120.

With reference now to Figure 2, illustrated is an aspect of the medical sensing communication system 100. Specifically, Figure 2 is a diagrammatic perspective view of the bedside utility box (BUB) 101 of Figure 1. As mentioned above, the BUB 101 is a hub through which the medical sensing-related tools communicate with data network 128. In general, the BUB 101 is operable to collect medical sensing data from connected medical sensing devices such as the IVUS PIM 112 and OCT PIM 114 and send it to remote computing resources to be processed. Once processed, the medical sensing data may be returned to the BUB 101, where it is routed to the control surfaces 118 and 120 to be displayed and analyzed by clinicians.

The BUB 101 includes a number of sockets to which the medical sensing-related tools connect. In the illustrated embodiment, the BUB 101 includes an ECG socket 130, an auxiliary power socket 132, a FLIVUS foot switch socket 134, a FLIVUS PIM socket 136, an OCT PIM socket 138, two USB sockets 140 and 142, a display socket 144, a FFR PIM socket 146, a IVUS PIM socket 148, and a network communication socket 150. Referring to Figures 1 and 2, the ECG device 116 may couple to the ECG socket 130, the IVUS PIM 112 may couple to the IVUS PIM socket 148, the bedside controller 118 may couple to the USB socket 140, and the control room control surface may couple to the USB socket 142. The USB sockets 140 and 142 to which the control surfaces 118 and 120 connect may alternatively be replaced with other short-distance high-speed ports such as FireWire ports or Thunderbolt ports. Further, in some embodiments, the boom display 122 may be coupled to the display socket 144, which, in the current embodiment, is a VGA port, but may alternatively be a DVI port, S-video port, DisplayPort port, HDMI port, or other type of video display port. Additionally, the BUB 101 may communicate with the network 128 through the network communication socket 150. In the illustrated embodiment, the network communication socket 150 is an Ethernet port, but alternatively, it may be another type of network communication port such as a fiber optic port. Or, if the network 128 is a data bus network, the network communication socket 150 may be a USB port, a InfiniBand port, a HyperTransport port, a Thunderbolt port, FireWire port, or other data bus port. In the illustrated embodiment, the sockets of BUB 101 providing connectivity to medical sensing-related tools are dedicated to a specific medical sensing modality and thus conform to a number of different form factors. However, in alternative embodiments, the sockets may be substantially similar (i.e. are standardized). Further, although the BUB 101 includes certain sockets for specific medical sensing-related tools, in other embodiments, it may include additional and/or different sockets to connect medical sensing devices, controllers, and displays.

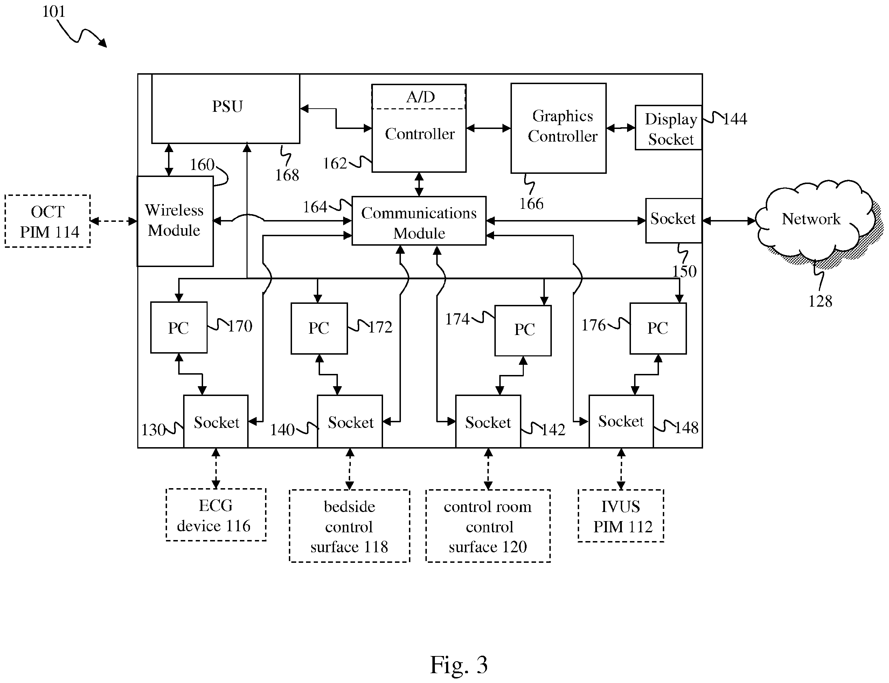

Figure 3 is a functional block diagram of an embodiment of a bedside utility box (BUB). As described in Figure 2, the BUB 101 may include the ECG socket 130, USB socket 140 and 142, and the IVUS PIM socket 148, to which the ECG device 116, bedside control surface 118, control room control surface 120, and IVUS PIM 112 respectively connect. The BUB 101 also includes the display socket 144 and network communication socket 150, to which the boom display 122 and network 128 respectively connect. For purposes of clarity, the other sockets of BUB 101 illustrated in Figure 2 have been omitted from the functional block diagram of Figure 3. The BUB 124 further includes a wireless communication module 160 operable to communicate with medical sensing-related tools in close proximity to the BUB, such as the OCT PIM 114. In one embodiment, the wireless communication module 160 may be a wireless personal area network (WPAN) communication module such as an Ultra-wide band (UWB) module, a wireless FireWire module, or wireless USB module or some other high-speed wireless module.

The BUB 101 further includes a controller 162 and a communication module 164. In some embodiments, the controller 162 may be a low-power microcontroller with integrated memory and peripherals (i.e. a system-on-a-chip). However, in other embodiments, the controller may be a general-purpose, central processing unit (CPU). The controller 162 (i.e. control module) is operable, among other things, to route data from the sockets 130, 140, 142, 148 and the wireless communication module 160 to the communication module 164, where it may be transmitted to the network 128 via socket 150. In the current embodiment, the controller 162 includes an analog to digital (A/D) converter which the controller may selectively utilize based on whether data incoming from a connected PIM is in analog or digital form. For example, the controller 162 may convert analog data from a PIM to digital data before it is routed to the communication module 164. Additionally, in some embodiments, the controller 162 may be operable to associate identifying information with the medical sensing data as it is routed to the communications module 164. More specifically, the controller 162 may create a plurality of digital messages from the incoming analog or digital data stream, where each message contains a portion of the digitized medical sensing data and a header. The aforementioned U.S. Provisional Patent Application No. 61/473,591, entitled "Distributed Medical Sensing System and Method," discloses creating messages that associate identifying information with medical sensing data in more detail. Further, in the event that multiple medical sensing devices are coupled to the BUB 101, as illustrated in Figure 3, the controller 162 may be operable to facilitate time synchronization among the devices for co-registration purposes. For instance, in one embodiment, the controller 162 may be operable to serve as a master time server for the PIMs 112 and 114 using a network-based time synchronization protocol such as the Precision Time Protocol (PTP) or the Network Time Protocol (NTP). In another embodiment, the controller 162 may be operable to assign a common timestamp to data as it arrives into the BUB 101 from a plurality of medical sensing devices. Further, in another embodiment, the controller 162 may communicate with connected medical sensing devices using a synchronous protocol such as Synchronous Optical Networking (SONET), and may assign timestamps to incoming medical sensing data based on the multiplexed communication. Still further, in other embodiments, the BUB 101 may include a dedicated real-time clock to synchronize sampling by connected medical sensing devices. In such an embodiment, the real-time clock may distribute a synchronization signal to connected sensing devices and also the controller 162 which may act as a co-registration processor. In some embodiments, the real-time clock may be integrated into the controller 162. The aforementioned U.S. Provisional Patent Application No. 61/473,591, entitled "Distributed Medical Sensing System and Method," discloses temporally synchronizing medical sensing data collection in more detail.

Further, in some embodiments, the controller 162 may be operable to modify the medical data received from the medical sensing devices as it is routed to the communication module 164. For example, in some embodiments, the controller 162 may compress the data before it is transmitted over the network 128. In this manner, large data sets produced by imaging modalities such as OCT may be more efficiently moved over the network 128. In some embodiments, the controller 162 may also be operable to filter incoming sensing data in some manner.

The communication module 164 in the BUB 101 is a high-speed communication module operable to transmit data received from the medical sensing-related tools connected to the BUB 101 and the network 128. In embodiments in which the network 128 is packet-based, the communication module 164 is operable to packetize medical sensing data routed by (and possibly digitized by) the controller 162, address the resulting packets, and the send the packets out over the network 128. In the embodiments in which the controller 162 segments incoming sensing data into messages, the communication module 164 may encapsulate the messages into TCP/IP packets for transmission over the network 128. In the illustrated embodiment, the communication module 140 is an Ethernet-based communication module, however, in other embodiments the communications module may be a InfiniBand switched fabric communications module, HyperTransport communication module, a fiber optic link module, a USB controller, a Thunderbird controller, a FireWire controller, a high-speed wireless module or some other high-speed communication module known in the art.

The BUB 101 further includes a graphics controller 166 operable to output images to the display socket 144. In the illustrated embodiment, the graphics controller 166 is a graphics processing unit (GPU) independent of the controller 162, but, in other embodiments, it may be integrated into the controller 162 or be a plug-in, off-the-shelf component. Further, in some embodiments, the BUB 101 may include a plurality of display sockets, where the graphics controller 166 is capable outputting distinct video signals to each. The graphics controller 166 gives the BUB 101 the capability to independently output images representative of medical sensing data received from the connected medical sensing devices. In some embodiments, this capability may be utilized to reduce network bandwidth requirements. For example, data associated with a medical sensing modality that requires minimal processing (e.g. FFR) may be processed by controller 162 and immediately output to a display via graphics controller 166, thereby eliminating the need to send the data over the network 128 to be processed. Further, to mitigate against lost connectivity with network 128 during a catheterization procedure, the graphics controller 166 may be used to perform some rudimentary image processing of incoming medical sensing data. For example, if during an OCT procedure in which the BUB 101 is receiving OCT data from OCT PIM 114, the computing resources on network 128 become unavailable, the controller 162 and graphics controller 166 may be operable to perform some minimal image processing on the OCT data and output rudimentary OCT images to the display boom 122. Further, in some embodiments, the BUB 101, upon receiving medical sensing data, may render basic image data with graphics controller 166 and simultaneously transmit an unprocessed version of the medical data to computing resources on network 126 for more advanced processing and storage. A software framework executing on the controller 162 in the BUB 101 manages the routing, analog to digital conversion, and image processing of incoming medical sensing data. This software framework will be discussed in greater detail in association with Figure 4.

The BUB 101 further includes a medical-grade power supply unit (PSU) 168. The PSU 168 provides power to the controller 162, wireless module 160, and the medical sensing-related tools (e.g. medical sensing devices, control surfaces) connected to the sockets 130, 140, 142, and 148. As mentioned above, different connected tools may have different power requirements. For example, a phased-array catheter for use in IVUS procedures may require more power than a pressure sensor mounted on the distal end of a guide wire for use in a FFR procedure. Thus, in the current embodiment, the PSU 168 is a multi-stage power supply that includes an isolating transformer to step down an input AC voltage and a plurality of switching power converters (PC) 170, 172, 174, 176 to dynamically output a desired voltage at a specific socket. For example, the PSU 168 may step down a 120V AC supply to an intermediate DC voltage and then each power converter (i.e. power module) may convert the intermediate voltage to one of a plurality of DC voltages such as 48V, 24V, 12V, 5V, and 3.3V. The specific voltage output to each socket dynamically depends on the specific power requirements of the medical sensing-related tool connected. In one embodiment, the PCs 170, 172, 174, 176 may be physically integrated into the PSU 168. In other embodiments, the PSU 168 may be a different type of power supply known in the art and power conversion and distribution may be done by other methods known by those of ordinary skill in the art. Additionally, upon connection of a medical sensing-related device to one of the sockets 130, 140, 142, and 148, the controller 162 is operable to automatically interrogate the tool to determine connection attributes such as voltage and communication protocol. In the current embodiment, the controller 162 utilizes a low-voltage (e.g. 5V) TTL logic initialization process to communicate with a newly connected medical sensing tool. In alternative embodiments, however, a different type of initialization process may be used. After the initialization process is complete, the controller 162 is operable to dynamically set the voltage output to the tool and, in some embodiments, the protocol with which the tool communicates with the BUB 101.

Note that the functional block diagram shown in Figure 3 has been simplified for the sake of clarity. A person of ordinary skill in the art would understand that elements of the BUB 101 may be rearranged or combined and that additional elements may be added without changing the functionality described herein. Further, a person of ordinary skill in the art understands that in the context of the current disclosure, a module may refer to a hardware module, a software module, or a combination software and hardware module.

With reference now to Figure 4, illustrated is a functional block diagram of the software framework executing on the BUB 101. More specifically, Figure 4 illustrates one embodiment of the medical sensing communication system 100 in which the IVUS PIM 112, the OCT PIM 114, an angiography system 180, the bedside controller 118, the boom display 122, and data network 128 are communicatively coupled to the BUB 101. As mentioned above, the BUB 101 includes a software framework to route, digitize, and process medical data received from connected medical sensing devices. The software framework includes a plurality of software layers that manage various aspects of the BUB 101. For instance, an operating platform 182 undergirds the software framework and provides the core functionality of the BUB 101. For instance, the operating platform 182 may manage power consumption and distribution of the BUB 101 and may also manage network connectivity. Further, the software framework may include an analog to digital conversion engine 184 operable to digitize analog data incoming from medical sensing devices, also may include a video engine 186 operable to render images and video associated with medical sensing data. Additionally, the software framework includes a routing manager 188 operable to control the routing of data between the medical sensing-related tools connected to BUB 101 and the network 128. In some embodiments, the routing manager may be operable to create a plurality of messages from digitized sensing data, where each message includes identifying information about the associated data.

Each of the software layers 182, 184, 186, and 188 additionally expose application programming interfaces (APIs) through which system resources may be accessed. The software framework in BUB 101 includes a processing application layer 190 in which processing applications associated with specific medical sensing modalities may execute. Utilizing the APIs exposed by the underlying software layers, the processing applications in the application layer 190 may be operable to render video or other images (e.g. FFR signal traces) from the raw medical sensing data transmitted to the BUB 101 by connected medical sensing devices. For example, if the BUB 101 loses network connectivity with the network 128, an IVUS processing application in the application layer 190 may call video processing APIs exposed by the video engine 186 to render IVUS video images for display on the boom display 122. Further, in some embodiments, the application layer 190 may include co-registration applications, in which medical sensing data from a plurality of medical sensing devices is co-registered and processed to produce co-registration images for display via the boom monitor 122. For instance, a co-registration application may display an electrocardiogram (ECG) wave adjacent to IVUS imaging data. In an exemplary embodiment, additional processing applications may be added to the application layer 190 to support new medical sensing modalities or co-registration techniques developed after the BUB 101 has been deployed. Further, the API-based software framework allows the applications 190 to be independent of the supporting software layers and thus written by third parties to control custom workflows.

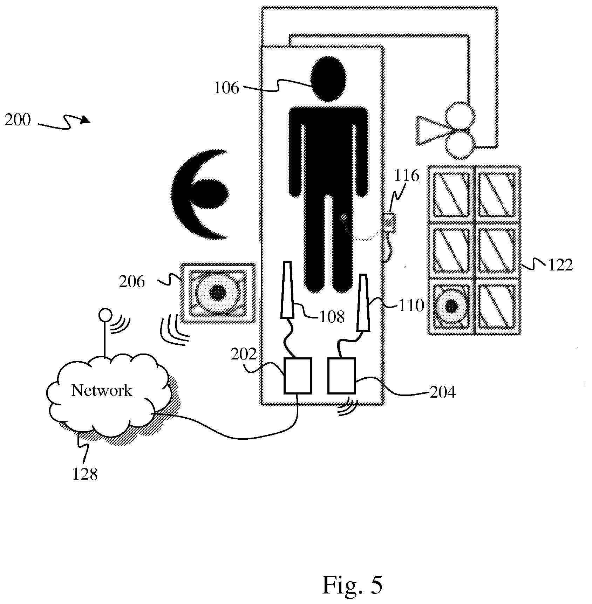

Referring now to Figure 5, shown is a schematic drawing depicting a medical sensing communication system 200 according to another embodiment of the present disclosure. The medical sensing communication system 200 is similar to the medical sensing communication system 100 shown in Figure 1. Like medical sensing communication system 100, medical sensing communication system 200 may be utilized to collect data from medical sensing devices in catheter room 102 and transmit it to remote computing resources, where it is processed and returned. However, in system 200, medical sensing devices collect and transmit medical sensing data to remote computing resources without the use of a bedside utility box (BUB).

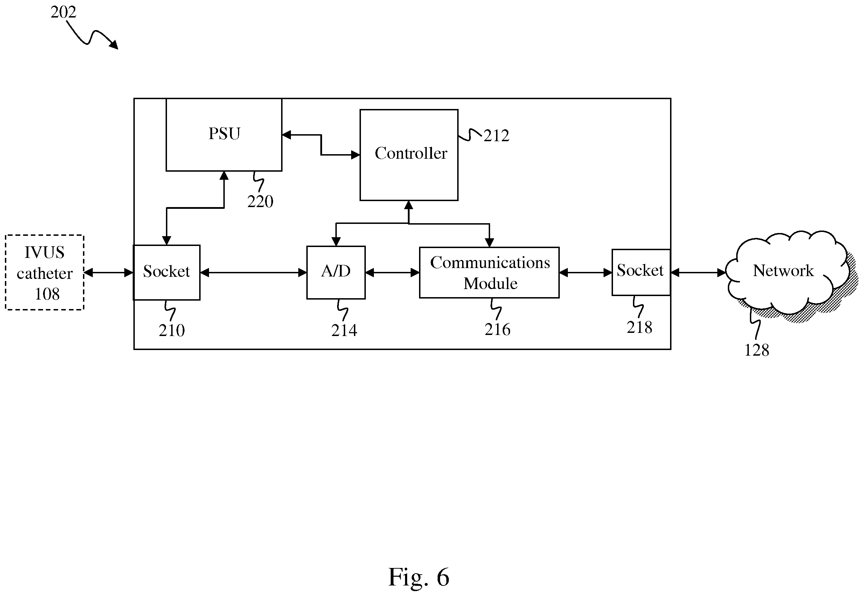

In more detail, the patient 106 may be undergoing a multi-modality procedure, in which IVUS data will be collected with the IVUS catheter 108 and OCT data will be collected with the OCT catheter 110. In the system 200, the IVUS catheter 108 is communicatively coupled to an IVUS PIM 202. As mentioned above, the IVUS catheter 108 may be capable of multi-modality sensing such as IVUS and IVPA, and PIM 202 may likewise capable of receiving multi-mode sensor outputs. Without a BUB in system 200, the IVUS PIM 202 may itself be considered a patient communication system that is operable to receive medical sensing data collected by the IVUS catheter 108 and transmit the received data onto the data network 128. As such, the PIM 202 is operable to perform similar functions that the PIM 112 and BUB 101 performed together in the patient communication system of Figure 1. As shown, IVUS PIM 202 is coupled to the IVUS catheter 108 via a wired connection. In alternative embodiments, however, the PIM-catheter connection may be wired or wireless. In the illustrated embodiment, the PIM 202 includes an analog to digital (A/D) converter and transmits digital data to the network 128 via a wired connection. The IVUS PIM 202 will be described in greater detail in association with Figure 6.

The medical sensing communication system 200 further includes a OCT PIM 204 configured to receive OCT data from the OCT catheter 110. Like the IVUS PIM 202, the OCT PIM 204 may itself be considered a patient communication system in the absence of a BUB. The OCT PIM 204 is operable to receive medical sensing data collected by the OCT catheter 110 and transmit the received data onto the data network 128. As shown, OCT PIM 204 is coupled to the OCT catheter 110 via a wired connection. In alternative embodiments, however, the PIM-catheter connection may be wired or wireless. In the illustrated embodiment, the PIM 204 includes transmits digital data to the network 128 via a wireless connection, such as an IEEE 802.11 Wi-Fi connection or another high-speed wireless connection.

The medical sensing communication system 200 further includes a bedside control surface 206. The bedside control surface 206 may be similar to the bedside control surface 118 of Figure 1 in that it provides user control and displays images of the particular medical sensing modality (or modalities) being used to diagnose the patient 106. However, in the current embodiment, the bedside control surface 206 communicates with the network 128 via a wireless connection, such as an IEEE 802.11 Wi-Fi connection or another high-speed wireless connection. The bedside control surface 206 is operable to wirelessly transmit workflow control information to the PIMs 202 and 204 via the network 128 and also receive medical sensing data from the PIMs that has been processed by remote computing resources on the network 128. Because the bedside control surface 206 is wireless, it may be moved to a control room, different catheter room, or even a doctor's office as needed. Although two PIMs are depicted as part of system 200, additional PIMs associated with different medical sensing modalities may be integrated in to system 200 and communicatively coupled to the network 128 without the use of a BUB.

Figure 6 is a functional block diagram of an embodiment of the IVUS PIM 202 of Figure 5. As described in Figure 5, the PIM 202 is communicatively coupled to both the IVUS catheter 108 and the network 128 and is operable to route medical sensing data from the catheter to computing resources on the network. The PIM 202 includes a socket 210 to which the catheter 108 connects. The socket 210 may be a dedicated IVUS port or may be a standardized port such that any catheter with a matching standardized connector may connect to it. The PIM 202 further includes a controller 212 operable to route data from the socket 210 through an A/D converter 214 and to a communications module 216. In some embodiments, the controller 212 may be similar to the low power controller 162 in the BUB 101 of Figure 3. As the PIM 202 is often placed near or on a patient during a procedure, it ideally gives off little or no heat in some embodiments. Alternatively, in some embodiments, the controller 212 is coupled to a heat sink that disperses heat generated by the controller. Alternatively, the BUB 101 may include active cooling elements to prevent heat buildup. Further, the controller 212 is operable to digitize analog data received from the IVUS catheter 108 with the A/D converter 214. Although, the A/D converter is depicted as an independent hardware module, in some embodiments, the A/D converter may be a software module executing on the controller 212. Additionally, in some embodiments, the data received from a catheter may already be digitized and thus controller 212 may disable the A/D converter 214.

Further, after incoming medical sensing data has been digitized, the controller 212 routes it to the communication module 216, which may be similar to the communication module 164 in the BUB 101 of Figure 3. In some embodiments, the controller 212 may be operable to associate identifying information with the medical sensing data as it is routed to the communications module 216. More specifically, the controller 212 may create a plurality of digital messages from the incoming analog or digital data stream, where each message contains a portion of the digitized medical sensing data and a header. The aforementioned U.S. Provisional Patent Application No. 61/473,591, entitled "Distributed Medical Sensing System and Method," discloses creating messages associating identifying information with medical sensing data in more detail. Further, in some embodiments, the controller 212 may be operable to modify the medical data received from the medical sensing devices as it is routed to the communication module 216. For example, in some embodiments, the controller 212 may compress the data before it is transmitted over the network 128. In some embodiments, the controller 212 may also be operable to filter incoming sensing data in some manner.

In embodiments in which the network 128 is packet-based, the communication module 216 is operable to packetize medical sensing data routed by (and possibly digitized by) the controller 212, address the resulting packets, and the send the packets out over the network 128 via a socket 218. In the embodiments in which the controller 212 segments incoming sensing data into messages, the communication module 212 may encapsulate the messages into TCP/IP packets for transmission over the network 128. In some embodiments, the PIM 202 may wirelessly transmit unprocessed medical sensing data to the network 128 like PIM 204, and, as such, the communication module 216 may be a wireless communication module. The PIM 202 further includes a medical-grade power supply unit (PSU) 220. The PSU 220 provides power to the controller 212, wireless module 216, and the phased-array transducers disposed on catheter 108 via the catheter's connection to socket 210. In some embodiments, the PSU 220 may be able to dynamically deliver varying amounts of power to socket 210 based on the type of catheter coupled to socket 210. For instance, the PSU may include a secondary-stage switching power converter similar to the power converters in the BUB 101 of Figure 3.

Although illustrative embodiments have been shown and described, a wide range of modification, change, and substitution is contemplated in the foregoing disclosure and in some instances, some features of the present disclosure may be employed without a corresponding use of the other features. For example, in some embodiments, the medical sensing communication system 100 may be used to process non-cardiovascular diagnostic data such data from cranial or peripheral arteries, as well as data from non-vascular body portions. Further, the system 100 may be used to collect and process MRI data, or may be utilized in computer assisted surgery (CAS) applications. It is understood that such variations may be made in the foregoing without departing from the scope of the present disclosure. Accordingly, it is appropriate that the appended claims be construed broadly and in a manner consistent with the scope of the present disclosure.

## Claims

1. A method of collecting medical sensing data with a patient communication system (101) including a controller (162) disposed within a housing of the patient communication system, the method comprising:
receiving first medical data associated with a first modality from a first medical sensing device (108) communicatively coupled to the controller via a first socket;
receiving second medical data associated with a second modality from a second medical sensing device communicatively coupled to the controller via a second socket, the second modality being different than the first modality;
the controller digitizing the first and second medical data if it is received in analog form;
a network communication module (164) disposed within the housing transmitting the digitized first and second medical data onto a data network (128);
the controller automatically communicating with the first and second medical sensing device upon connection to determine a power requirement of the first and second medical sensing device; and
providing a power supply unit (168) including an isolating transformer for stepping down an input AC voltage and outputting a dynamical amount of power to a first and second power module (174, 176) to dynamically output a desired voltage at the first and second socket, respectively;
the first power module (176) disposed within the housing providing a first dynamic amount of power to the first medical sensing device through the first socket, and
the second power module (176) disposed within the housing providing a second dynamic amount of power to the second medical sensing device through the second socket, wherein the second dynamic amount of power is different than the first dynamic amount of power,
wherein the first and second dynamic amount of power is based on the power requirement of the first and second medical sensing device, respectively.

2. The method of claim 1, further including the controller automatically communicating with the first medical sensing device upon connection to determine a communication protocol of the first medical sensing device.

3. The method of claim 1 or claim 2, wherein the automatically communicating includes communicating with the first medical sensing device using a low-voltage TTL logic initialization process.

4. The method of claim 1, further including the controller processing the first medical data using a first processing application associated with the first modality to produce first processed medical data.

5. The method of claim 4, further including a graphics controller (166) disposed within the housing and communicatively coupled to the controller outputting the first processed medical data to a display.

6. The method of claim 1, further including the controller processing the first medical data using a first processing application associated with the first modality to produce first processed medical data, and the controller processing the second medical data using a second processing application different from the first processing application and associated with the second modality to produce second processed medical data.

7. The method of claim 1, further including processing the first and second medical data using a co-registration processing application to produce co-registered medical data.

8. The method of claim 1, wherein the first medical data includes one of intravascular ultrasound (IVUS) imaging data, optical coherence tomography (OCT) data, fractional flow reserve (FFR) data, forward looking IVUS (FLIVUS) imaging data, and intravascular photoacoustic (IVPA) imaging data.

9. The method of claim 1, further including:
receiving the first medical data from the first medical sensing device at a first patient isolation module (112) communicatively interposed between the first medical sensing device and the controller, the first patient isolation module being operable to transmit the first medical data to the controller and to route power from the first power module to the first medical sensing device.

10. The method of claim 1, wherein the first medical sensing device includes a catheter configured to enter into a vessel of a body.

11. The method of claim 1, wherein the second medical data is received wirelessly.

12. The method of claim 1, wherein the network communication module is wirelessly communicatively coupled to the data network, and wherein the network communication module transmitting the digitized second medical data includes the network communication module wirelessly transmitting the digitized second medical data to the data network.

## Patentansprüche

1. Verfahren zum Sammeln von medizinischen Messdaten mit einem Patientenkommunikationssystem (101), das eine Steuerung (162) einschließt, die innerhalb eines Gehäuses des Patientenkommunikationssystems angeordnet ist, wobei das Verfahren umfasst:
Empfangen von ersten medizinischen Daten, die mit einer ersten Modalität assoziiert sind, von einer ersten medizinischen Messvorrichtung (108), die über eine erste Buchse kommunikationsmäßig mit der Steuerung gekoppelt ist;
Empfangen von zweiten medizinischen Daten, die mit einer zweiten Modalität assoziiert sind, von einer zweiten medizinischen Messvorrichtung, die über eine zweite Buchse kommunikationsmäßig mit der Steuerung gekoppelt ist, wobei sich die zweite Modalität von der ersten Modalität unterscheidet;
Digitalisieren, durch die Steuerung, der ersten und zweiten medizinischen Daten, wenn dieselben in analoger Form empfangen werden;
Übertragen, durch ein Netzwerkkommunikationsmodul (164), das innerhalb des Gehäuses angeordnet ist, der digitalisierten ersten und zweiten medizinischen Daten zu einem Datennetzwerk (128);
automatisches Kommunizieren der Steuerung mit der ersten und zweiten medizinischen Messvorrichtung bei Verbindung, um einen Strombedarf der ersten und zweiten medizinischen Messvorrichtung zu bestimmen; und
Bereitstellen einer Stromversorgungseinheit (168), die einen Trenntransformator einschließt, zum Abwärtswandeln einer AC-Eingangsspannung und Ausgeben einer dynamischen Strommenge an ein erstes und zweites Strommodul (174, 176), um eine gewünschte Spannung dynamisch an jeweils die erste und zweite Buchse auszugeben;
wobei das erste Strommodul (176), das innerhalb des Gehäuses angeordnet ist, der ersten medizinischen Messvorrichtung durch die erste Buchse eine erste dynamische Strommenge bereitstellt, und
wobei das zweite Strommodul (176), das innerhalb des Gehäuses angeordnet ist, der zweiten medizinischen Messvorrichtung durch die zweite Buchse eine zweite dynamische Strommenge bereitstellt, wobei sich die zweite dynamische Strommenge von der ersten dynamischen Strommenge unterscheidet,
wobei die erste und zweite dynamische Strommenge auf dem Strombedarf jeweils der ersten und zweiten medizinischen Messvorrichtung basiert.

2. Verfahren nach Anspruch 1, das weiter das automatische Kommunizieren der Steuerung mit der ersten medizinischen Messvorrichtung bei Verbindung einschließt, um ein Kommunikationsprotokoll der ersten medizinischen Messvorrichtung zu bestimmen.

3. Verfahren nach Anspruch 1 oder Anspruch 2, wobei das automatische Kommunizieren das Kommunizieren mit der ersten medizinischen Messvorrichtung unter Verwendung eines Niederspannungs-TTL-Logikinitialisierungsprozesses einschließt.

4. Verfahren nach Anspruch 1, das weiter das Verarbeiten der ersten medizinischen Daten durch die Steuerung unter Verwendung einer ersten Verarbeitungsanwendung einschließt, die mit der ersten Modalität assoziiert ist, um erste verarbeitete medizinische Daten zu erzeugen.

5. Verfahren nach Anspruch 4, das weiter das Ausgeben, durch eine Grafiksteuerung (166), die innerhalb des Gehäuses angeordnet und kommunikationsmäßig mit der Steuerung gekoppelt ist, der ersten verarbeiteten medizinischen Daten an eine Anzeige einschließt.

6. Verfahren nach Anspruch 1, das weiter das Verarbeiten der ersten medizinischen Daten durch die Steuerung unter Verwendung einer ersten Verarbeitungsanwendung, die mit der ersten Modalität assoziiert ist, um erste verarbeitete medizinische Daten zu erzeugen, und das Verarbeiten der zweiten medizinischen Daten durch die Steuerung unter Verwendung einer zweiten Verarbeitungsanwendung, die sich von der ersten Verarbeitungsanwendung unterscheidet und mit der zweiten Modalität assoziiert ist, einschließt, um zweite verarbeitete medizinische Daten zu erzeugen.

7. Verfahren nach Anspruch 1, das weiter das Verarbeiten der ersten und zweiten medizinischen Daten unter Verwendung einer Co-Registrierungs-Verarbeitungsanwendung einschließt, um co-registrierte medizinische Daten zu erzeugen.

8. Verfahren nach Anspruch 1, wobei die ersten medizinischen Daten eines einschließen aus Intravaskulärer Ultraschall (IVUS) -Bildgebungsdaten, Optische Kohärenztomographie (OCT) -Daten, Fraktionelle Flussreserve (FFR) -Daten, Forward Looking IVUS (FLIVUS) -Bildgebungsdaten, und Intravaskuläre Photoakustik (IVPA) - Bildgebungsdaten.

9. Verfahren nach Anspruch 1, das weiter einschließt:
Empfangen der ersten medizinischen Daten von der ersten medizinischen Messvorrichtung an einem ersten Patienten-Trennmodul (112), das kommunikationsmäßig zwischen der ersten medizinischen Messvorrichtung und der Steuerung eingefügt ist, wobei das erste Patienten-Trennmodul so betrieben werden kann, dass es die ersten medizinischen Daten an die Steuerung überträgt, und Strom vom ersten Strommodul zur ersten medizinischen Messvorrichtung routet.

10. Verfahren nach Anspruch 1, wobei die erste medizinische Messvorrichtung einen Katheter einschließt, der dazu konfiguriert ist, in ein Gefäß eines Körpers einzudringen.

11. Verfahren nach Anspruch 1, wobei die zweiten medizinischen Daten drahtlos empfangen werden.

12. Verfahren nach Anspruch 1, wobei das Netzwerkkommunikationsmodul kommunikationsmäßig drahtlos mit dem Datennetzwerk gekoppelt ist, und wobei das Übertragen der digitalisierten zweiten medizinischen Daten durch das Netzwerkkommunikationsmodul das drahtlose Übertragen der digitalisierten zweiten medizinischen Daten durch das Netzwerkkommunikationsmodul an das Datennetzwerk umfasst.

## Revendications

1. Procédé de recueil de données de détection médicales avec un système de communication au patient (101) incluant un dispositif de commande (162) disposé dans un boîtier du système de communication au patient, le procédé comprenant :
la réception de premières données médicales associées à une première modalité venant d'un premier dispositif de détection médical (108) couplé en communication avec le dispositif de commande via une première douille ;
la réception de secondes données médicales associées à une seconde modalité venant d'un second dispositif de détection médical couplé en communication avec le dispositif de commande via une seconde douille, la seconde modalité étant différente de la première modalité ;
le dispositif de commande numérisant les premières et secondes données médicales si elles sont reçues sous forme analogique ;
un module de communication en réseau (164) disposé dans le boîtier et transmettant les premières et secondes données médicales numérisées sur un réseau de données (128) ;
le dispositif de commande communiquant automatiquement avec le premier et le second dispositif de détection médical lors de la connexion pour déterminer un besoin en énergie du premier et du second dispositif de détection médical ; et
la fourniture d'une unité d'alimentation électrique (168) incluant un transformateur isolant pour abaisser une tension d'entrée CA et délivrer une quantité dynamique d'énergie à un premier et un second module de puissance (174, 176) pour délivrer de manière dynamique une tension souhaitée au niveau de la première et de la seconde douille, respectivement ;
le premier module de puissance (176) disposé dans le boîtier fournissant une première quantité dynamique d'énergie au premier dispositif de détection médical via la première douille et
le second module de puissance (176) disposé dans le boîtier fournissant une seconde quantité dynamique d'énergie au second dispositif de détection médical via la seconde douille, dans lequel la seconde quantité dynamique d'énergie est différente de la première quantité dynamique d'énergie,
dans lequel la première et la seconde quantité dynamique d'énergie sont basées sur le besoin en énergie du premier et du second dispositif de détection médical, respectivement.

2. Procédé selon la revendication 1, incluant en outre le dispositif de commande qui communique automatiquement avec le premier dispositif de détection médical lors de la connexion pour déterminer un protocole de communication du premier dispositif de détection médical.

3. Procédé selon la revendication 1 ou la revendication 2, dans lequel la communication automatique inclut la communication avec le premier dispositif de détection médical en utilisant un processus d'initialisation logique à basse tension TTL.

4. Procédé selon la revendication 1, incluant en outre le dispositif de commande qui traite les premières données médicales en utilisant une première application de traitement associée à la première modalité pour produire des premières données médicales traitées.

5. Procédé selon la revendication 4, incluant en outre un dispositif de commande graphique (166) disposé dans le boîtier et couplé en communication avec le dispositif de commande qui délivre les premières données médicales traitées à un appareil d'affichage.

6. Procédé selon la revendication 1, incluant en outre le dispositif de commande qui traite les premières données médicales en utilisant une première application de traitement associée à la première modalité pour produire des premières données médicales traitées et le dispositif de commande qui traite les secondes données médicales en utilisant une seconde application de traitement différente de la première application de traitement et associée à la seconde modalité pour produire des secondes données médicales traitées.

7. Procédé selon la revendication 1, incluant en outre le traitement des premières et secondes données médicales en utilisant une application de traitement en co-enregistrement pour produire des données médicales co-enregistrées.

8. Procédé selon la revendication 1, dans lequel les premières données médicales incluent l'une parmi des données d'imagerie ultrasoniques intravasculaires (IVUS), des données de tomographique à cohérence optique (OCT), des données de réserve d'écoulement fractionnée (FFR), des données d'imagerie prospectives IVUS (FLIVUS) et des données d'imagerie photo-acoustiques intravasculaires (IVPA).

9. Procédé selon la revendication 1, incluant en outre :
la réception des premières données médicales venant du premier dispositif de détection médical sur un premier module d'isolement de patient (112) intercalé en communication entre le premier dispositif de détection médical et le dispositif de commande, le premier module d'isolement de patient étant à même de transmettre les premières données médicales au dispositif de commande et d'acheminer de l'énergie du premier module de puissance au premier dispositif de détection médical.

10. Procédé selon la revendication 1, dans lequel le premier dispositif de détection médical inclut un cathéter configuré pour pénétrer dans un vaisseau d'un corps.

11. Procédé selon la revendication 1, dans lequel les secondes données médicales sont reçues sans fil.

12. Procédé selon la revendication 1, dans lequel le module de communication de réseau est couplé en communication sans fil au réseau de données et dans lequel le module de communication de réseau transmettant les secondes données médicales numérisées inclut le module de communication de réseau qui transmet sans fil les secondes données médicales numérisées au réseau de données.
